(19) Europäisches Patentamt
European Patent Office
Office européen des brevets

(11) EP 1 083 186 B1

(12) **EUROPÄISCHE PATENTSCHRIFT**

(45) Veröffentlichungstag und Bekanntmachung des Hinweises auf die Patenterteilung:
**28.01.2009 Patentblatt 2009/05**

(51) Int Cl.:
*C08F 4/00* (2006.01) *C08F 2/38* (2006.01)

(21) Anmeldenummer: **00118125.4**

(22) Anmeldetag: **28.08.2000**

(54) **Oligomere und polymere Telechelen**

Telechelic oligomers and polymers

Oligomères et polymères téléchéliques

(84) Benannte Vertragsstaaten:
**DE ES FR GB IT NL**

(30) Priorität: **07.09.1999 DE 19942614**
**07.09.1999 DE 19942615**

(43) Veröffentlichungstag der Anmeldung:
**14.03.2001 Patentblatt 2001/11**

(73) Patentinhaber: **Bayer MaterialScience AG**
**51368 Leverkusen (DE)**

(72) Erfinder:
- **Melchiors, Martin, Dr.**
  **51373 Leverkusen (DE)**
- **Höcker, Hartwig, Prof. Dr.**
  **52076 Aachen (DE)**
- **Keul, Helmut, Dr.**
  **52074 Aachen (DE)**
- **Achten, Dirk**
  **52066 Aachen (DE)**

(56) Entgegenhaltungen:
**WO-A-97/46593** **WO-A-98/31739**

- **HAWKER, CRAIG J. ET AL: "Radical Crossover in Nitroxide Mediated "Living" Free Radical Polymerizations" , J. AM. CHEM. SOC. (1996), 118 (46), 11467-11471 XP000960412 * Seite 11468; Beispiel SCHEME2 * * Seite 11469; Beispiel SCHEME5 ***
- **HAWKER, CRAIG J.: "Architectural control in "living" free radical polymerizations: preparation of star and graft polymers" , ANGEW. CHEM., INT. ED. ENGL. (1995), 34(13/14), 1456-9 XP000960411 * Beispiele SCHEME1,2,4 ***
- **JAHN, ULLRICH: "Highly efficient generation of radicals from ester enolates by the ferrocenium ion. Application to selective.alpha.-oxygenation and dimerization reactions of esters" , J. ORG. CHEM. (1998), 63(21), 7130-7131 XP000960555 See entire doct.**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 083 186 B1

## Beschreibung

Die Erfindung betrifft Telechele auf Basis von Vinylpolymerisaten, ein Verfahren zu deren Herstellung sowie ihre Verwendung im Kunststoff-, Faser- oder Lackbereich.

Als Telechele werden im allgemeinen lineare Oligomere oder niedermolekulare lineare Polymere mit funktionellen Gruppen an beiden Kettenenden bezeichnet; eine umfassende Übersicht über die Herstellung von Telechelen findet man in Adv. Polym. Sci., 1987, 81, 168. Sie haben u. a. Bedeutung als Additive und als Bausteine (Pre-polymere) für höhermolekulare Copolymere definierter Struktur (z.B. Blockcopolymere, Kammpolymere, Sternpolymere) erlangt. Insbesondere für die Verwendung von Telechelen als Bausteine für Copolymere ist eine möglichst exakte Bifunktionalität notwendig. Die bekanntesten Reaktionen zur Herstellung von Telechelen, die eine exakte Funktionalität von 2 aufweisen, sind Polyadditionsreaktionen (z.B. zu Polyurethanen, Polyharnstoffen), Polykondensationen (z.B. zu Polyestern, Polycarbonaten, Polyamiden) sowie ringöffnende anionische oder kationische Polymerisationen von heterocyclischen Monomeren (z.B. cyclische Ester, Carbonate, Acetale oder Ether), ggf. mit Abbruchreagenzien, die die gewünschten funktionellen Gruppen enthalten.

Für eine Verwendung in der Lackindustrie sind telechele Polyacrylate, d.h. nieder-molekulare Acrylatcopolymere mit zwei definierten funktionellen Endgruppen, die an den üblicherweise in der Lackchemie verwendeten Vernetzungs-,Kettenverlängerungs-und/oder Kopplungsreaktionen teilnehmen können, von großem Interesse.

Mit keinem der oben beschriebenen Verfahren zur Herstellung von Telechelen lassen sich jedoch telechele Polyacrylat-Copolymere herstellen.

In der Polymerchemie sind verschiedene Methoden bekannt, um Polyvinyl- oder Polyacrylatverbindungen mit funktionellen Endgruppen zu versehen. Oxidative Kettenspaltungen (z.B. mit Sauerstoff, Ozon sowie Osmium- oder Rutheniumtetroxid) verlaufen unspezifisch und/oder setzen Doppelbindungen in den Polymerketten als Angriffspunkt der Spaltung voraus. Eine exakte Bifunktionalität läßt sich so kaum erreichen. Setzt man in eine radikalische Polymerisation einen auf die Funktionalität zwei berechneten Anteil Monomere, die die gewünschte fünktionclle Gruppe tragen, zu, so erhält man ein Produktgemisch mit einen mittleren Funktionalität von zwei, in dem bifunktionelle Moleküle neben tri- und höherfunktionellen, monofunktionellen aber auch nichtfunktionellen Polymermolekülen vorliegen. Dies beruht auf dem statistischen Charakter der radikalischen Polymerisation und auf einem schwer kontrollierbaren Einfluß der verschiedenen Abbruchreaktionen

Setzt man anstelle der funktionelle Gruppen tragenden Monomere Initiatoren und/oder Abbruchreagenzien ein, die die gewünschten funktionellen Gruppen tragen (z.B. funktionalisierte Diazoverbindungen, funktionalisierte Peroxide oder Redox-Initiatoren), so wird eine Funktionalität von 2 im allgemeinen deshalb nicht erreicht, weil sich das Verhältnis der in Konkurrenz auftretenden Abbruchreaktionen Disproportionierung, Rekombination bzw. Abbruch durch Initiatorradikale oder Abbruchreagenz nicht gezielt steuern läßt. Bei der sog. "Dead end - Polymerisation" setzt man einen so großen Überschuß eines die gewünschte Endgruppe tragenden Initiators ein, daß praktisch jede Polymerkette mit einem Initiatormolekül abgebrochen wird und somit bifunktionell ist; man erreicht so allerdings nur sehr niedrige Molekulargewichte und die entstehenden Produkte werden aufgrund der großen Initiatormengen unwirtschaftlich teuer.

Bei der Telomerisation, d.h. der Polymerisation von Vinyl- oder Acrylatmonomeren in Gegenwart von Kettenübertragungsreagenzien mit hohen Kettenübertragungskonstanten, erreicht man ebenfalls nur geringe Molekulargewichte, und die Anwendung bleibt auf wenige Fälle beschränkt (z.B. Polymerisation in Gegenwart von Atetrachlorkohlenstoff, Dibrommethan oder funktionelle Gruppen tragenden Disulfiden). Da sich die Disproportionierung als Abbruchreaktion zwischen zwei aktiven Kettenenden nicht ganz unterdrücken läßt, findet man Funktionalitäten der Telechele kleiner 2. Zumindest im Falle der Halogenverbindungen ist auch eine nachträgliche polymeranaloge Umsetzung der Halogensubstituenten zu den gewünschten funktionellen Gruppen notwendig

Telechele Polymethacrylate können durch Gruppentransferpolymerisation mit Kettensilylacetalen hergestellt werden, wobei die funktionellen Endgruppen durch Umwandlung der Silylgruppen gebildet werden. Nachteilig sind hier aber die hohen Reinheitsanforderungen an Monomer und Lösemittel sowie Preis und Verfügbarkeit der benötigten Initiatoren, wodurch ein solches Verfahren nur für Spezialanwendungen anwendbar wäre.

Aus der EP-A 0 613 910 und EP-A0 622 378 ist bekannt, $\alpha,\omega$-Polymethacrylatdiole durch selektive Umesterung der terminalen Estergruppe eines $\alpha$-hydroxyfunktionellen Polyalkylmethacrylats herzustellen. Dieses Verfahren weist mehrere Nachteile auf. Zum einen stellt man das ($\alpha$-hydroxyfunktionelle Polyalkylmethacrylat durch radikalische Polymerisation in Gegenwart großer Mengen Mercaptoethanol her, was mit erheblicher Geruchsbelästigung verbunden ist. Zum anderen handelt es sich um einen mehrstufigen, energie- und zeitaufwendigen Prozess, der das Abdestillieren des Überschusses Mercaptoethanol und des verwendeten Lösemittels, die Umesterung mit einem überschuß eines Diols in Gegenwart eines Katalysators, die destillative Entfernung des Methanols, mehrmaliges Waschen des Produkts zur Entfernung des Katalysators und des überschüssigen Diols sowie noch weitere Reinigungsstufen umfasst. Außerdem bleibt diese Reaktion auf die ausschließliche Verwendung von Alkylmethacrylaten beschränkt, da sonst die Umesterungsreaktion nicht mehr ausreichend selektiv an der terminalen Estergruppe der Kette abläuft.

Einen Spezialfall ohne Anwendungsbreite und wirtschaftliches Potential stellt auch die ringöffnende Polymerisation ungesättigter Heterocyclen dar (z.B. cyclische Kettenacetale, ungesättigte Spiroorthocarbonate); solche Monomere sind technisch nicht verfügbar.

In der der Publikation von Craig J. Hawker et al "Radical Crossover in Nitroxide Mediated "Living" Free Radical Polymerizations", J. Am. Chem. Soc. (1996), 118(46), 11467-11471, werden monofunktionelle Telechele (Schema 2 = HOST) mit einem Funktionalisierungsgrad kleiner als 1 beschrieben, wobei die funktionelle Gruppe durch verschiedene Modifizierungen in weitem Bereich wählbar ist. Dabei handelt es sich nicht um Initiatoren des HOSTOH-Typs; die die Herstellung von Isocyanatgruppenreaktiven bifunktionellen Telechelen erlauben würden. Es handelt sich vielmehr um Telechele mit einem TEMPO-Molekül als Endgruppe, welche nicht mit z.B. Isocyanatgrüppen im Sinne einer Additionsreaktion reagieren können, sondern nur über fortschreitende radikalische/thermische Aktivierung zur weiteren Reaktionen fähig sind. Somit ist keine der bisher aufgeführten Methoden zur Herstellung der gewünschten telechelen Polyacrylate geeignet, da entweder die angestrebte Funktionalität nicht erreicht wird, die Methode nur auf einige wenige Spezialfälle beschränkt bleibt und/oder polymeranaloge Nachreaktionen erforderlich sind. Es ist ein Polymerisationsverfahren erforderlich, das bei einfacher Durchführbarkeit eine gute Kontrolle der Polymerisation und insbesondere der Endgruppen der Polymerketten ermöglicht. Ein solches Verfahren ist die lebende radikalische Polymerisation.

Die lebende radikalische Polymerisation stellt eine relativ junge Methode der kontrollierten radikalischen Polymerisation dar. Sie verbindet die Vorteile einer konventionellen radikalischen Polymerisation (einfaches Syntheseverfahren, kostengünstig, breite Monomerbasis) mit denen einer lebenden Polymerisation (Polymere mit definiertem Aufbau, / Molekulargewicht und -verteilung und Endgruppenfunktionalität). Das Ziel einer genauen Kontrolle der radikalischen Polymerisation wird hier durch einen reversiblen Kettenabbruch bzw. Blockierung ("end-capping") nach jedem Wachstumsschritt erreicht. Die Gleichgewichtskonzentration der polymerisationsaktiven Kettenenden ist dabei im Vergleich zur Gleichgewichtskonzentration der blockierten ("dormant") Kettenenden so gering, daß Abbruch- und Übertragungsreaktionen gegenüber der Wachstumsreaktion stark zurückgedrängt sind. Da das End-capping reversibel abläuft, bleiben alle Kettenenden "lebend", sofern kein Abbruchreagenz vorhanden ist. Dies ermöglicht die Kontrolle des Molekulargewichts, einen niedrigen Polymolekularitätsindex und gezielte Funktionalisierung der Kettenenden durch Abbruchreagenzien.

Erste Ansätze für eine kontrollierte radikalische Polymerisation unter Verwendung von Teraalkylthiuramdisulfiden werden bei Otsu et al. (Makromol. Chem., Rapid Commun. 1982, 3, 127-132) beschrieben. Die Herstellung von Telechelen, deren funktionelle Gruppen zu einer weiteren Umsetzung oder Vernetzung mit in der Lackchemie gebräuchlichen funktionellen Gruppen fähig sind, wird hier nicht beschrieben.

Einen anderen Lösungsweg bietet die ATRP (Atom Transfer Radical Polymerization), bei der eine Übergangsmetall-Komplexverbindung $ML_x$ ein übertragbares Atom oder Atomgruppe X (z.B. Cl, Br) aus einer organischen Verbindung RX abstrahiert unter Bildung einer oxidierten Komplexverbindung $ML_xX$ und eines organischen Radikals R●, das sich an ein Vinylmonomer Y unter Bildung des Kohlenstoffradikals RY● addiert. Dieses Radikal kann mit der oxidierten Komplexverbindung unter Übertragung von X zu RYX und $ML_x$ abreagieren, welches eine neue ATRP und damit einen weiteren Wachstumsscbritt auslösen kann. Die polymerisationsaktive Spezies RY● ist somit durch die abstrahierbare Gruppe X mit Hilfe der Übergangsmetallverbindung, die den Redoxprozeß ermöglicht, reversibel blockiert.

Dieser Ansatz wird u.a. von Sawamoto et al (M = Ru, X = Cl; Macromolecules 1995, 282 1721; Macromolecules 1996, 29, 1070), Percec et al. (M = Cu, RX =Arylsulfonylhalogenid); Macromolecules 1995 28, 7970), der Fa. Du Pont (M= Co (u.a.), R● aus R-N=N-R; WO 95/25765) und insbesondere von Matyjaszewski et al. (WO 96/30421, WO 97/18247) beschrieben. In den letztgenannten Dokumenten werden auch (Co)polymere mit ein oder zwei funktionellen Endgruppen beschrieben, wobei diese Endgruppen polymeranalog aus den zunächst immer vorliegenden Halogenid-Endgruppen gebildet werden. Dieses Verfahren weist aber den Nachteil auf, daß zur Herstellung der gewünschten Telechele nach der eigentlichen Polymerisationsreaktion noch ein oder mehrere Reaktionsschritte notwendig sind, um die Halogenidgruppen zu den gewünschten funktionellen Gruppen umzuwandeln, wobei andere Gruppen, wie z.B. die Estergruppen der Acrylatmonomere, unangetastet bleiben müssen. Außerdem weist das ATRP-Verfahren den Nachteil auf, daß die Polymere durch ein aufwendiges Reinigungsverfahren vom verwendeten Katalysatorsystem (Cu, Bipyridin) abgetrennt werden müssen. Rückstände von Cu beeinträchtigen die Färbung und das Gebrauchsverhalten der so erhaltenen Polymere.

Aus der US-A 4581429 sind Alkoxyamine bekannt, die durch Reaktion von linearen oder cyclischen Nitroxiden wie z.B. 2,2,6,6-Tetramethylpiperidin-1-oxyl (TEMPO) mit organischen Kohlenstoff-basierten Radikalen gebildet werden, sowie ein Verfahren zur Herstellung von Vinylpolymeren unter Verwendung dieser Initiatoren. Bei Temperaturen >100°C kann die C-ON -Bindung reversibel unter Rückbildung des C-Radikals *("active species")* und des stabilen Nitroxidradikals gespalten werden. Das Gleichgewicht liegt hierbei weit auf der Seite des Alkoxyamins (*"dormant species").* Ergebnis dieser Reaktion ist eine geringe stationäre Radikalkonzentration, die bei der radikalischen Polymerisation von Vinylmonomeren dazu führt, daß bimolekulare Abbruchreaktionen gegenüber der unimolekularen Wachstumsreaktion kinetisch benachteiligt sind. So werden Nebenreaktionen weitgehend unterdrückt und eine "lebende" Reaktionsführung bei der radikalischen Polymersation möglich. Hydroxyfunktionelle Endgruppen werden ebenfalls beschrieben und hier durch polymeranaloge reduktive Abspaltung der TEMPO-Endgruppen mit Zn/Essigsäure erreicht.

Die Herstellung von Vinylpolymeren durch lebende radikalische Polymerisation ("**S**table **F**ree **R**adical **P**ölymerization", SFRP) auf Basis von Alkoxyaminen beschreiben Hawkeret al. (J.Am.Chem.Soc. 1994, 116, 11185; Macromolecules 1995, 28, 2993) sowie *Georges et al.* (Xerox Comp., US 5322912, US 5401804, US 5412047, US 5449724, WO 94/11412, WO 95/26987, WO 95/31484). Die Kohlenstoff Radikale werden dabei durch Anlagerung von Radikalstartern (Peroxide, Azoinitiatoren) an radikalisch polymerisierbare Monomere hergestellt; diese Radikale werden dann in situ von TEMPO zu Alkoxyaminen abgefangen. Diese Alkoxyamine stellen die eigentlichen Initiatoren dar, da sie bei Temperaturen >100°C reversibel in Radikale gespalten werden und so die Polymerisation der zudosierten Monomere starten können. Bei der Polymerisation ist dann die Zahl der wachsenden Ketten (und damit das Molekulargewicht) durch die Konzentration der Alkoxyamin-Initiatoren bestimmt. Dieses Polymerisationsverfahren bietet gegenüber der ATRP den Vorteil der Metallfreiheit, d.h. der aufwendige Schritt der Abtrennung des Cu-Katalysators und seiner Umsetzungsprodukte entfällt hier. Difunktionelle Telechele sind bislang nur durch kettenanaloge Umsetzungen (oxidative Abspaltung der Tempo Endgruppe) erhalten worden. Die Synthese von Polyacrylat-Telechelen, deren funktionelle Gruppen zu einer weiteren Umsetzung oder Vernetzung mit in der Lackchemie gebräuchlichen funktionellen Gruppen fähig sind, durch Anwendung difunktioneller Alkoxyamininitiatoren wurde bisher nicht beschrieben.

Die WO 97/46593 beschreibt die Herstellung von hydrbxytelechelen Butadienpolymeren durch SFRP, Die Polymerisation von Butadien erfolgt in Anweisenheit von $H_2O_2$ und TEMPO in einem polaren Lösungsmittel. $H_2O_2$ reagiert als Starter sowie als Terminierungsreagenz. Erhalten werden Oligomere <3000 mit einem Polymolekularitätsindex von 1,3 - 3,4 und OH-Funktionalitäten von 0,59 - 1,69. Die Verwendung von funktionalisierten Alkoxyamin-Initiatoren und/oder Acrylatmonomeren oder Styrol ist hier nicht beschrieben.

Die Verwendung von Alkoxyamin-Initiatoren, die zusätzlich funktionelle Gruppen tragen, die zu einer weiteren Umsetzung oder Vernetzung mit in der Lackchemie gebräuchlichen funktionellen Gruppen fähig sind, zur Herstellung telecheler Polyacrylat-Copolymere ist in keinem der aufgeführten Dokumente oder Verfahren des Stands der Technik beschrieben.

Aufgabe der vorliegenden Erfindung war daher die Bereitstellung eines Verfahrens zur Herstellung von oligomeren und polymeren Telechelen HO-B'-Q-B" oder HO-B'-Q-C-B"' das die o.g. Nachteile des Standes der Technik nicht aufweist. Insbesondere wird also nach einem Verfahren gesucht, das es erlaubt, in einfacher Weise möglichst in einem Reaktionsschritt ohne nachträgliche Aufreinigung ein Homo- oder Copolymer aus einem oder mehreren Vinylmonomeren, insbesondere Acrylatmonomeren und Styrol, mit gezielt eingestelltem Molekulargewicht und niedrigem Polymolekularitätsindex herzustellen und mit zwei funktionellen Endgruppen (Y, OH) zu funktionalisieren, wobei Y eine mit Isocyanaten, Alkoholen, Carbonsäuren, Anhydriden und/oder Epoxiden reaktionsfähige funktionelle Gruppe darstellt. Das erhaltene Polymere soll darüberhinaus eine zur Verarbeitung ausreichende thermische Stabilität von ≥ 200°C aufweisen.

Diese Aufgabe konnte gelöst werden durch eine radikalische (Co)polymerisation olefinisch ungesättigter Monomere der Formel

CHR'=CR"R"'

worin

R', R", R"' unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, auch weitere Substituenten wie z.B. Ethergruppen enthalten können und auch Bestandteil einer Ringstruktur, z.B. bei einem cyclischen Anhydrid, Ester, Amid oder Kohlenwasserstoff sein können, nach der Methode der "lebenden" radikalischen Polymerisation SFRP in Gegenwart eines funktionalisierten Alkoxyamin-mitiators der allgemeinen Strukturformel **I**:

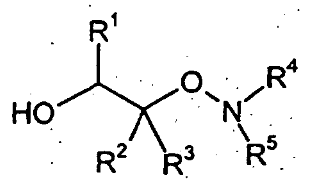

(I)

wobei

[-$CR^1$-$CR^2R^3$-] durch B' dargestellt wird,

$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, und

[-O-$NR^4R^5$] durch B" bzw. B"' dargestellt wird,

$R^4$, $R^5$ unabhängig voneinander aliphatische, cycloaliphatische oder gemischt aliphatisch/aromatische Reste mit 1 - 24 Kohlenstoffatomen, die auch Teil eines 4-8-gliedrigen Rings sein können, darstellen, worin das dem Alkoxyamin-Stickstoffatom direkt benachbarte Kohlenstoffatom der Reste $R^4$ und $R^5$ jeweils mit 3 weiteren organischen Substituenten (d.h. nicht Wasserstoff) oder einem doppelt gebundenen Kohlenstoff-, Sauerstoff-, Schwefel- oder Stickstoffatom und einem weiteren organischen Substituenten (nicht Wasserstoff) substituiert ist, und worin Im Fall von B''' mindestens einer der Reste $R^4$, $R^5$ eine mit Isocyanaten, Alkoholen, Carbonsäuren, Anhydriden und/oder Epoxiden reaktionsfähige funktionelle Gruppe Y enthält, gegebenenfalls in Gegenwart von bzw. durch nachträgliche Zugabe eines Funktionalisierungsreagenzes, das nach Verbrauch der Monomere die Ketten abbricht und das mindestens eine C=C-Doppelbindung und eine mit Isocyanaten, Alkoholen, Carbonsäuren, Anhydriden und/oder Epoxiden reaktionsfähige funktionelle Gruppe Y trägt.

Die nach diesem Verfahren erhaltenen Polymere tragen 2 endständig eingebaute funktionelle Gruppen: ein Kettenende ist mit der OH-Gruppe des Alkoxyamin-Initiators substituiert [-HO-B'-]; die zweite funktionelle Gruppe Y wird entweder über das Funktionalisierungsreagenz (Variante 1, monofunktionalisierter Alkoxyamin-Initiator [-C-B''']) oder über den Alkoxyamin-Initiator (Variante 2, difunktionalisierter Alkoxyamin-Initiator [-B"]) endständig in das Polymer eingeführt

Weiterer Gegenstand der Erfindung sind Telechele auf Basis von Vinyl-Polymerisaten des Molekulargewichts $200 < M_n < 50000$ und der Funktionalität von 1,5 bis 2,0 und einem Polymolekularitätsindex (im Bereich von $1,1 < Mw/Mn < 1,8$, engthaltend eine Hydroxyl-Endgruppe und eine mit Isocyanaten, Alkoholen, Carbonsäuren, Anhydriden und/oder Expoxiden reaktionsfähige funktionelle Gruppen Y.

Gegenstand der Erfindung sind auch Kunststoffe, Klebstoffe oder Fasern sowie Bindemittel, Bindemittelkomponenten, Härter oder Härterkomponenten enthaltend die erfindungsgemäßen Telechelen oder deren Reaktionsprodukte.

Das erfindungsgemäße Verfahren umfasst zwei Varianten, die zu den gewünschen Telechelen HO-B'-Q-B" oder HO-B'-Q-C-B''' führen.

Das erfindungsgemäße Verfahren nach Variante 1 beinhaltet [HO-B'-Q-C-B'''] die (Co)-Polymerisation oder -Oligomerisation von geeigneten Monomeren A durch einen monofunktionalisierten Alkoxyamin-Initiator B der allgemeinen Strukturformel **I**, der - außer seiner OH-Gruppe - keine weitere mit Isocyanaten, Alkoholen, Carbonsäuren, Anhydriden und/oder Epoxide reaktionsfähige funktionelle Gruppe Y enthält, und wobei die Reste $R^1$ bis $R^5$ ansonsten die bei Formel **I** genannte Bedeutung haben, gefolgt von einer Endgruppenfunktionalisierung dieses Polymers durch ein geeignetes Funktionalisierungsreagenz C nach vollständigem Umsatz der Monomere.

Das Reagenz C trägt dabei eine mit Isocyanaten, Alkoholen, Carbonsäuren, Anhydriden und/oder Epoxiden reaktionsfähige funktionelle Gruppe Y und kann während der Polymerisation zugegen sein oder dem Ansatz nach weitgehend vollständigem Verbrauch der Monomere zugegeben werden. Es bricht die ein radikalisches Kettenende tragenden Polymerketten nach vollständigem Umsatz der Monomere ab und führt so zu einer Endgruppenfunktionalisierung des zweiten Kettenendes mit der funktionellen Gruppe Y.

Y ist ausgewählt aus der Gruppe der funktionellen Einheiten mit den allgemei- nen Formeln -OH, -CN, -COOH, $-COOR^{18}$, -SH, $-NHR^{18}$, $-OCONHR^{19}NCO$, $-[O-CH_2-CHR^{20}-]_m-OH$ sowie

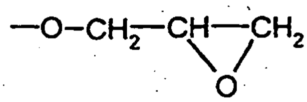,

wobei

$R^{16}$ Wasserstoff oder eine lineare oder verzweigte $C_1$-$C_6$-Alkylkette und

$R^{18}/R^{19}$ beliebige $C_1$-$C_{24}$-Kohlenwasserstoffreste darstellen und

$R^{20}$ Wasserstoff oder ein $C_1$-$C_{24}$-Alkylrest und

m 1 bis 10 sein kann

Das erfindungsgemäße Verfahren nach Variante 2 [HO-B'-Q-B"]beinhaltet die Verwendung difunktionalisierten Alkoxyamin-Initiatoren der allgemeinen Strukturformel, **I**, wobei die Reste $R^1$ bis $R^5$ die o.g. Bedeutung haben; diese Alkoxyamin-Initiatoren enthalten jedoch - im Unterschied zu Variante 1 - zwingend eine mit Isocyanaten, Alkoholen, Carbonsäuren, Anhydriden und/oder Epoxiden reaktionsfähige funktionelle Gruppe Y. Bei dieser Variante wird ohne Einsatz eines zusätzlichen Funktionalisierungsreagenzes C ein Telechel der gewünschten Struktur erhalten.

Erfindungsgemäß können bei den radikalisch polymerisierbaren Monomeren A - prinzipiell alle aus dem Stand

der Technik bekannten radikalisch polymerisierbaren Olefine und substituierten Olefine eingesetzt werden. Als Substituenten kommen z.B. in Frage:

- H,
- lineare oder verzweigte Alkylreste mit 1 - 20 Kohlenstoffatomen, die gewebenenfalls auch weitere Substituenten tragen können,
- $\alpha,\beta$-ungesättigte lineare oder verzweigte Alkenyl- oder Alkinylreste, die gegebenfalls auch weitere Substituenten tragen können,
- Cycloalkylreste, die auch Heteroatome wie z.B. O, N oder S im Ring und gegebenenfalls weitere Substituenten tragen können,
- gegebenenfalls substituierte Aryl- oder Heteroarylreste,
- Halogen, CN, $CF_3$, COOR, COR.

Die radikalisch polymerisierbare Doppelbindung kann auch Teil eines Rings sein, wie z.B. bei cyclischen Olefinen oder olefinisch ungesättigten Anhydriden, Estern, Amiden oder Imiden.

Bevorzugt eingesetzte Monomere umfassen: (Meth)acrylsäureester von $C_1$-$C_{20}$-Alkoholen, Acrylnitril, Cyanoacrylsäureester von $C_1$-$C_{20}$=Alkoholen, Maleinsäurediester von $C_1$-$C_6$-Alkoholen, Maleinsäureanhydrid, Vinylpyridine, Vinyl(alkylpyrrole), Vinyloxazole, Vinyloxazoline, Vinylthiazole,Vinylimidazole, Vinylpyrimidine, Vinylketone, Styrol oder Styrolderivate, die in $\alpha$-Stellung einen $C_1$-$C_6$-Alkylrest oder Halogen tragen und bis zu 3 weitere Substituenten am aromatischen Ring tragen.

Besonders bevorzugt werden Butylacrylat, 2-Ethylhexylacrylat, Methylmethacrylat, Butylmethacrylat, Cyclohexylmethacrylat, Isobornylmethacrylat,Maleinsäureanhydrid oder Styrol eingesetzt. Es können auch beliebige Gemische der o.g. Monomeren eingesetzt werden.

Erfindungsgemäß können als Komponente B funktionalisierte Alkoxyamin-Initiatoren der allgemeinen Strukturformel **I** eingesetzt werden,

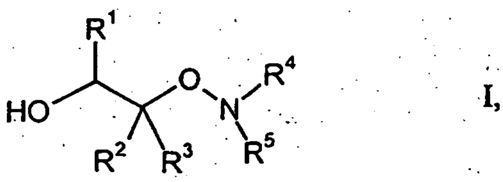

**I,**

wobei
$R^1$, $R^2$, $R^3$ $R^4$, und $R^5$ die o.g. Bedeutung haben, und worin mindestens einer der Reste $R^4$, $R^5$ eine mit Isocyanaten, Alkoholen, Carbonsäuren, Anhydriden und/oder Epoxiden reaktionsfähige funktionelle Gruppe Y enthalten kann.

Bevorzugt werden Alkoxyamin-Initiatoren der allgemeinen Strukturformel **II b**:

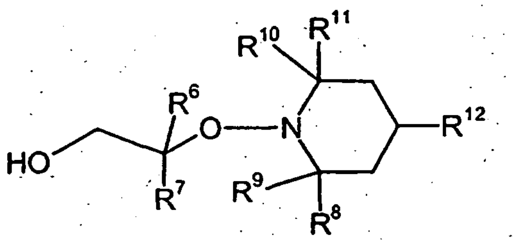

**II b,**

worin
$R^6$ = H oder $CH_3$ ist, $R^7$ einen ggf. substituierten Benzolrest oder eine Estergruppe der Formel -C(=O)O$R^{13}$ darstellt, worin $R^{13}$ eine (cyclo)aliphatische Alkylgruppe mit 1-20 Kohlenstoffatomen ist, $R^8$, $R^9$, $R^{10}$ und $R^{11}$ unabhängig voneinander $C_1$-$C_{20}$-(Cyclo)alkyl- oder $C_6$-$C_{24}$-Arylreste bzw. $C_7$-$C_{24}$-aliphafisch/aromatische Kohlenwasserstoffreste sein können, die zusätzlich Cyanogruppen, Ethergruppen, Amidgruppen oder OH-Gruppen enthalten können und auch Teil einer Ringstruktur sein können, und $R^{12}$ entweder Wasserstoff oder eine mit Isocyanaten, Alkoholen, Carbonsäuren, Anhydriden und/oder Epoxiden reaktionsfähige funktionelle Gruppe Y ist oder enthält, auf Basis von Acrylat- und Methacrylat-Monomeren, wie sie in der Lacktechnologie üblicherweise in Polyacrylat-(Co)polymeren verwendet werden, eingesetzt.

Besonders bevorzugt sind Alkoxyamin-Initiatoren der Strukturformel II c:

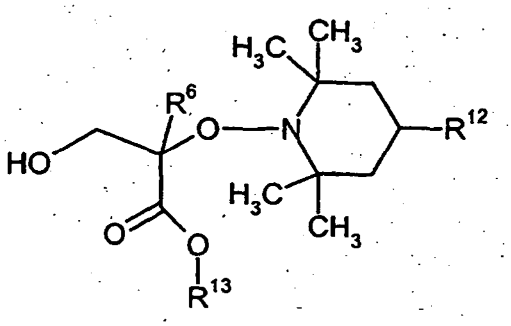

II c,

worin

$R^6$ = H oder $CH_3$ ist, $R^{12}$ = H oder eine der funktionellen Gruppen OH, $NH_2$ oder NHR ist, und $R^{13}$ = $CH_3$ oder $C_4H_9$ ist.

Ob der verwendete Alkoxyamin-Initiator B eine funktionelle Gruppe Y nach o.g. Definition trägt oder nicht, hängt davon ab, in welcher der beiden Verfahrensvarianten (s.o.) er eingesetzt wird. In Variante 1 verwendet man Alkoxyamin-Initiatoren B, die frei von funktionellen Gruppen Y nach o.g. Definition sind; in Variante 2 vierwendet man Alkoxyamin-Initiatoren B, die eine funktionelle Gruppe Y laut. o.g. Definition in einem der Reste $R^4$, $R^5$ oder $R^{12}$ enthalten.

Beim Funktionalisierungsreagenz C handelt es sich um eine Verbindung der Formel $R^{14}R^{15}C=CR^{16}(R^{17}\text{-}Y)$, die mindestens eine olefinische Doppelbindung und mindestens eine mit Isocyanaten, Alkoholen, Carbonsäuren, Anhydriden und/oder Epoxiden reaktionsfähige funktionelle Gruppe Y enthält, wobei zwischen der Doppelbindung und Y ein Kohlenstoffrest $R^{17}$ vorhanden sein muß, der eine lineare oder verzweigte, ggf. substituierte Alkylkette mit einer Mindestlänge von 1 Methylengruppe darstellt, und wobei $R^{14}$, $R^{15}$ und $R^{16}$ unabhängig voneinander Wasserstoff oder einen gegebenenfalls aryl- oder halogensubstituierten Alkylrest darstellen. In manchen Fällen, aber nicht bevorzugt, kann auch ein Gemisch solcher Verbindungen eingesetzt werden.

Bevorzugt wählt man die Komponente C aus Verbindungen der Gruppe

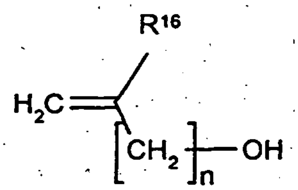
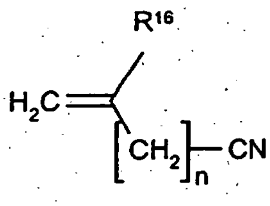
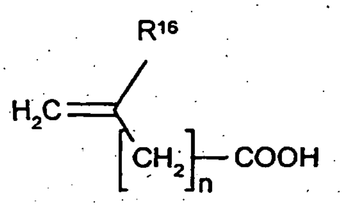

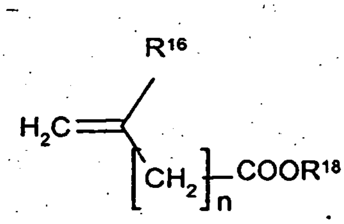
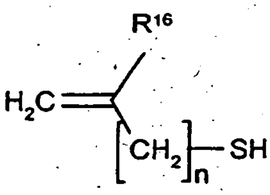
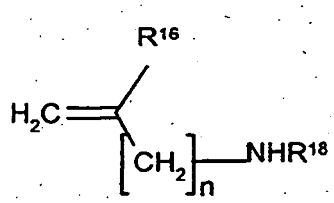

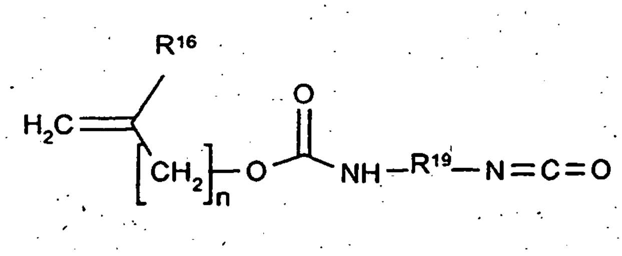

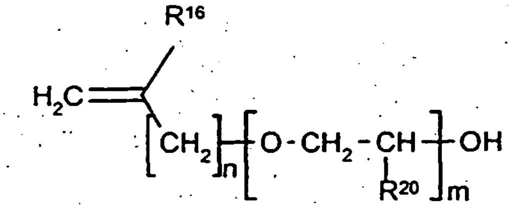

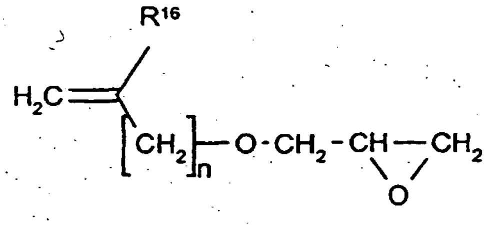

wobei
$R^{16}$ Wasserstoff oder eine lineare oder verzweigte $C_1$ - $C_6$-Alkylkette und R1[8] und $R^{19}$ beliebige $C_1$-$C_{24}$-Kohlenwasserstoffreste darstellen, $R^{20}$ Wasserstoff oder ein $C_1$-$C_{24}$-Alkylrest, bevorzugt Methyl ist, n = 1 - 4 und m = 1 - 10 sein kann, aus.

Besonders bevorzugt ist die Verwendung von 2-Propen-1-ol, 3-Buten-1-ol, 4-Penten-1-ol, 5-Hexen-1-ol oder ihrer durch Addition von 1 - 10 mol Propylenoxid an die OH-Gruppe erhaltenen propoxylierten Derivate.

Das Reagenz C kann während der Polymerisation zugegen sein oder dem Ansatz nach weitgehend vollständigem Verbrauch der Monomere zugegeben werden; es bricht die ein radikalisches Kettenende tragenden Polymerketten nach vollständigem Umsatz der Monomere ab und führt so zu einer Endgruppenfunktionalisierung des zweiten Kettenendes mit der funktionellen Gruppe Y.

Zur Herstellung der gewünschten Telechele nach dem erfindungsgemäßen Verfahren müssen die Komponenten Monomer und Alkoxyamin-Initiator und gegebenenfalls Funktionalisierungsreagenz in einem bestimmten Verhältnis vorliegen.

Das Verhältnis von Monomerem zum Alkoxyamin-Initiator richtet sich nach dem gewünschten Molekulargewicht bzw. Polymerisationsgrad des Telechels. Da es sich bei dem erfindungsgemäßen Verfahren um eine lebende Polymerisation handelt, die im Wesentlichen frei von Abbruch- oder Übertragungsreaktionen ist, und sich das gegebenenfalls eingesetzte Funktionalisierungsreagenz (aufgrund seiner im Vergleich zu den Monomeren deutlich geringeren Reaktionsgeschwindigkeit mit Radikalen) erst nach Verbrauch des Monomeren an die aktiven Kettenenden addiert, kann der Fachmann leicht die bei gegebener Ausgangsmonomerkonzentration [Mo] und erwünschtem Umsatz $x_p$ die erforderliche Initiatorkonzentration [I] berechnen, wenn er den Polymerisationsgrad $P_n$ erreichen will:

$$[I] = x_p * [M]_0 / P_n$$

wobei
$X_p = ([M]_0-[M]) / [M]_0$ den Umsatz und [M] die aktuelle Monomerkonzentration beim Umsatz $x_p$ darstellt. Hieraus wird ersichtlich, daß sich nach dem erfindungsgemäßen Verfahren Telechele beliebigen Molekulargewichts herstellen lassen. Bevorzugt werden aber Molekulargewichte von $200 < M_n < 50.000$, besonders bevorzugt Molekulargewichte von $500 < M_n < 10000$, ganz besonders bevorzugt von $1000 < M_n < 5000$ eingestellt. Die erhaltenen Polymolekularitätsindizes sind recht niedrig und liegen i.a. im Bereich von $1{,}1 < M_w/M_n < 1{,}8$, in den meisten Fällen im Bereich von $1{,}2 < M_w/M_n < 1{,}5$.

Wird ein Funktionalisierungsreagenz C verwendet (Variante 1), so setzt man dieses in einer solchen Menge ein, die einem molaren Verhältnis von C=C-Doppelbindungen im Funktionalisierungsreagenz C zum Alkoxyamin-Initiator B von mindestens 1:1, bevorzugt mindestens 5:1 entspricht.

Die erfindungsgemäßen Telechele weisen die allgemeine Formel HO-B'-Q-B'' oder HO-B'-Q-C-B''' auf, wobei Q einen gegebenenfalls substituierten Kohlenwasserstoffrest der Formel

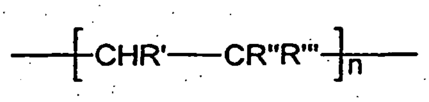

darstellt,
worin
n eine ganze Zahl im Bereich $3 \leq n \leq 500$ ist und worin R', R", R'" unabhängig voneinander H, $C_1$-$C_{20}$-(Cyclo)alkyl, $C_6$-$C_{24}$-Aryl, Halogen, CN, $C_1$-$C_{20}$-Alkylester oder -amid, $C_6$-$C_{24}$-Arylester oder -amid sein können, auch weitere Substituenten wie z.B. Ethergruppen enthalten können und auch Bestandteil einer Ringstruktur, z.B. bei einem cyclischen Anhydrid, Ester, Amid oder Kohlenwasserstoff sein können. Sie enthalten eine Hydroxygruppe als Endgruppe [HO-B'-], wobei B' ein Alkoxyamin-Fragment [$-CR_1$-$CR_2R_3$-] ist, in welchem
$R^1$, $R^2$, $R^3$ unabhängig voneinander H, $C_1$-$C_{20}$(Cyclo)alkyl, oder $C_6$-$C_{24}$-Aryl, Halogen, CN, $C_1$-$C_{20}$-(Cyclo)alkylester oder -amide, $C_6$-$C_{24}$-Arylester oder -amid können, sowie ein weiteres Alkoxyamin-Fragment [-B'' bzw. -B'''] der Formel

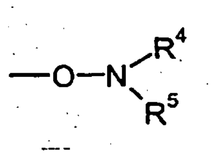

wobei
$R^4$, $R^5$ unabhängig voneinander aliphatische, cycloaliphatische oder gemischt alphatisch/aromatische Reste mit 1-24 Kohlenstoffatomen, die auch Teil eines 4-8-gliedrigen Rings sein können, darstellen, worin das dem Alkoxyamin-Stickstoffatom direkt benachbarte Kohlenstoffatom der Reste $R^4$ und $R^5$ jeweils mit 3 weiteren organischen Substituenten (d.h. nicht Wasserstoff) oder einem doppelt gebundenen Kohlenstoff-, Sauerstoff-, Schwefel- oder Stickstoffatom und einem weiteren organischen Substituenten (nicht Wasserstoff) substituiert ist, und worin im Fall von B'' mindestens einer der Reste $R^4$, $R^5$ eine mit Isocyanaten, Alkoholen, Carbonsäuren, Anhydriden und/oder Epoxiden reaktionsfähige funktionelle Gruppe Y enthält,
und gegebenenfalls ein Funktionalisierungsagenz C der Formel $R^{14}R^{15}C=CR^{16}(R^{17}$-Y),
wobei
$R^{14}$, $R^{15}$, $R^{16}$ unabhängig voneinander Wasserstoff oder einen gegebenenfalls aryl-oder halogensubstituituierten Alkylrest darstellen und
$R^{17}$ eine lineare oder verzweigte, gegebenenfalls substituierte Alkylkette mit einer Mindestlänge von 1 Methylengruppe darstellt.

Die erfindungsgemäßen Telechele weisen Funktionalitäten von 1,5 bis 2,0; bevorzugt jedoch von >1,8 bis 2,0; nie jedoch >2,0 auf. Eine der beiden Endgruppen kann auch in derivatisierter bzw. geschützter Form vorliegen, wobei sich dann Funktionalitäten von 0,7 bis 1,0; bevorzugt von > 0,8 bis 1,0 ergeben.

Die Umsetzung nach dem erfindungsgemäßen Verfahren kann bei Temperaturen zwischen Raumtemperatur und 180°C, bevorzugt zwischen 80° und 150°C, besonders bevorzugt zwischen 90° und 130°C durchgeführt werden. Sie kann lösemittelfrei (im Monomer bzw. Monomerengemisch) als auch in einem in der Lacktechnologie bekannten organischen Lösemittel durchgeführt werden. Sie kann an der Luft oder in einer Schutzgasatmosphäre durchgeführt werden; bevorzugt verwendet man eine Schutzgasatmosphäre (z.B. Stickstoff oder Argon).

Die erfindungsgemäßen Telechele HO-B'-Q-B'' oder HO-B'-Q-C-B''' können als Bausteine in Blockcopolymeren eingesetzt werden, die z.B. in Kunststoffen, Fasern, Klebstoffen oder Bindemitteln oder Bindemittelkomponenten in Beschichtungs-Mitteln enthalten sind. Je nach chemischer Natur der Kunstoffe, Fasern, Klebstoffe oder Bindemittel sowie der Funktionalitäten der übrigen darin enthaltenen Bausteine kann die funktionelle Gruppe Y des Telechels so gewählt werden, daß die Aufbaureaktionen zum Blockcopolymer leicht und kontrolliert ablaufen. Über die Monomerzusammensetzung des Mittelblocks Q des Telechels können in die daraus hergestellten Blockcopolymere Eigenschaften wie z.B. Härte, Flexibilität, Hydrophobie, Hydrophilie, gezielte Unverträglichkeiten oder zusätzliche Funktionalitäten gezielt eingeführt werden.

Die erfindungsgemäßen Telechele können auch unmodifiziert je nach Funktionalität Y als Bindemittel, Bindemittelkomponente, Härter oder Härterkomponente in Beschichtungsmitteln und Klebstoffen eingesetzt werden.

## Beispiele

Alle Angaben in % beziehen sich auf das Gewicht.

**Herstellung eines erfindungsgemäßen Telechels (Tabelle 1):**

In einen Glaskolben gibt man unter Stickstoffatmosphäre leq Alkoxyamin-Initiator und dazu x eq Monomeres (Varianten 1 und 2) und gegebenenfalls y eq Funktionalsierungsreagenz (Variante 1). Der Ansatz wird unter Stickstoff auf Reaktionstemperatur erhitzt und t h bei dieser Temperatur gerührt. Nach dieser Zeit wird Restmonomeres und ggf. Funktionalisierungsreagenz im Vakuum abgezogen.

Tabelle 1:

| Alkoxyamin-Initiator | Variante | Zeit t [h] | Temperatur [°C] | y = eq. Allylalkohol | x = eq. Monomer | Ausbeute [%] | $M_n$ (ber.)[1] | $M_n$ (GPC)[2] | $M_w/M_n$ (GPC)[2] | Funktionalität[3] a) | Funktionalität[3] b) |
|---|---|---|---|---|---|---|---|---|---|---|---|
| HOST | 1 | 50 | 130 | 5 | Styrol 40 | 82 | -- | 3700 | 1.18 | -- | 1,2 |
| HOST | 1 | 50 | 130 | 10 | Styrol 40 | 81 | -- | 3900 | 1.22 | -- | 1,5 |
| HOSTOH | 2 | 4 | 130 | 0 | Styrol 40 | 53 | 2460 | 2190 | 1,24 | > 1,9 | 1,85 |
| HOSTOH | 2 | 3 | 130 | 0 | styrol 20 MA 20 | 51 | 2110 | 2140 | 1,28 | > 1,9 | > 1,9 |
| HOSTOH | 2 | 3 | 130 | 0 | Styrol 20 / MMA 20 | 38 | 1660 | 1990 | 1,43 | 1,8 | >1,9 |
| HOSTOH | 2 | 4 | 130 | 0 | Styrol 20 / BuA 20 | 56 | 2760 | 2720 | 1,31 | 1,9 | 1,8 |
| HOSTOH | 2 | 4 | 130 | 0 | Styrol 20 / BuMA 20 | 41 | 2170 | 2400 | 1,49 | 1,7 | 1,9 |
| HOSTOH | 2 | 4 | 130 | 0 | Styrol 20 / t-BuA 20 | 52 | 2540 | 2420 | 1,23 | 1,9 | 1,8 |
| HOSTOH | 2 | 4 | 130 | 0 | Styrol 20 / t-BuMA 20 | 37 | 1930 | 2080 | 1,47 | 1,8 | > 1,9 |
| HOMATOH | 2 | 16 | 130 | 0 | Styrol 20 / t-BuA 20 | 46 | 2210 | 2500 | 1,34 | 1,7 | 1,9 |

Legende zu Tabelle 1:

[1] $M_n$ (ber.): Aus $[M]_0/[I]_0$ und dem Umsatz berechnetes Molekulargewicht

[2] GPC: Elutionsmittel THF; Polystyroleichung; $M_n$ und $M_w/M_n$ der erhaltenen Polymere.

[3] Funktionalität: bestimmt aus $^{31}$P-NMR-Spektren nach Funktionalisierung der Polymere mit Diphenylchlorophosphat;Funktionalisierung bezogen auf: a) $M_n$ (ber.); b) $M_n$ (GPC); Genauigkeit jeweils $\pm$ 0,1.

Verwendete Monomere: Styrol, Methylacrylat ***(MA)***, Methylmethacrylat (***MMA***), n-Butylacrylat (***BA***), *n*-Butylmethacrylat (***BMA***), *tert.*-Butylacrylat (***t-BA***), *tert.*-Butylmethacrylat ***(t-BMA)***.

Verwendete Alkoxyamininitiatoren:

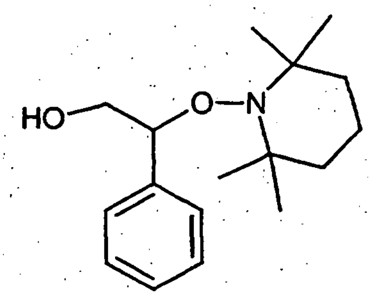

HOST

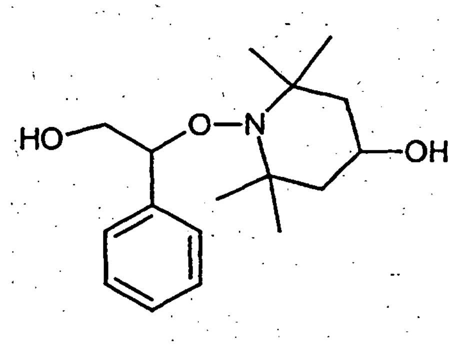

HOSTOH

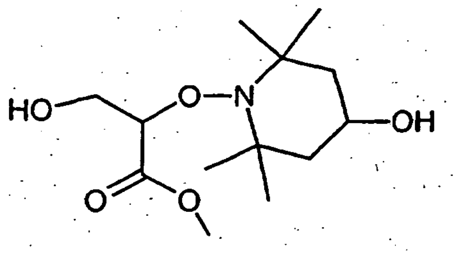

HOMATOH

## Patentansprüche

1. Verfahren zur Herstellung von Telechelen der Formel HO-B'-Q-B" oder HO-B'-Q-C-B'" des Molekulargewichts 500 < $M_n$ < 10000, wobei Q einen gegebenenfalls substituierten Kohlenwasserstoffrest der Formel

$$-\!\left[CHR'-CR''R'''\right]_n\!-$$

darstellt,
worin
n eine ganze Zahl im Bereich $3 \leq n \leq 500$ ist und worin R', R", R'" unabhängig voneinander H, $C_1$-$C_{20}$-(Cyclo)alkyl, $C_6$-$C_{24}$-Aryl, Halogen, CN, $C_1$-$C_{20}$-(Cyclo)Alkylester oder -amid, $C_6$-$C_{24}$-Arylester oder -amid sein können, auch weitere Substituenten wie Ethergruppen enthalten können und auch Bestandteil einer Ringstruktur, in einem cyclischen Anhydrid, Ester, Amid oder Kohlenwasserstoff sein können, **dadurch gekennzeichnet, daß** man

A) radikalisch polymerisierbare Monomere A, die keine mit Isocyanaten, Alkoholen, Carbonsäuren, Anhydriden und/oder Epoxiden reaktionsfähige funktionelle Gruppe Y enthalten, der Formel

CHR'=CR"R'"

mit
B) einem funktionalisierten Alkoxyamin-Initiator B der allgemeinen Strukturformel

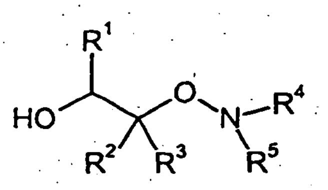

I

polymerisiert werden, wobei
[-$CR^1$-$CR^2R^3$-] durch B' dargestellt wird,
$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, und
[-$ONR^4R^5$] durch B" bzw. B"' dargestellt wird,
$R^4$, $R^5$ unabhängig voneinander aliphatische, cycloaliphatische oder gemischt aliphatisch/aromatische Reste mit 1-24 Kohlenstoffatomen, die auch Teil eines 4 - 8-gliedrigen Rings sein können, darstellen, worin das dem Alkoxyamin-Stickstoffatom direkt benachbarte Kohlenstoffatom der Reste $R^4$ und $R^5$ jeweils mit 3 weiteren organischen Substituenten (nicht Wasserstoff) oder einem doppelt gebundenen Kohlenstoff-, Sauerstoff-, Schwefel- oder Stickstoffatom und einem weiteren organischen Substituenten (nicht Wasserstoff) substituiert ist, und worin im Fall von B" mindestens einer der Reste $R^4$, $R^5$ eine mit Isocyanaten, Alkoholen, Carbonsäuren, Anhydriden und/oder Epoxiden reaktionsfähige funktionelle Gruppe Y enthält, gegebenenfalls in Gegenwart von bzw. durch nachträgliche Zugabe
C) eines Funktionalisierungsreagenzes C der allgemeinen Formel $R^{14}R^{15}C=CR^{16}(R^{17}\text{-}Y)$, wobei $R^{17}$ eine lineare oder verzweigte, gegebenenfalls substituierte Alkylkette mit einer Mindestlänge von 1 Methylengruppe darstellt, und wobei $R^{14}$, $R^{15}$ und $R^{16}$ unabhängig voneinander Wasserstoff oder einen gegebenenfalls aryl- oder halogensubstituierten Alkylrest darstellen.

2. Verfahren zur Herstellung von Telechelen gemäß Anspruch 1, **dadurch gekennzeichnet, daß** als Komponente A Butylacrylat, 2-Ethylhexylacrylat, Methylmethacrylat, Butylmethacrylat, Cyclohexylmethacrylat, Isobornylmethacrylat, Maleinsäureanhydrid oder Styrol bzw. ein Gemisch solcher Monomere eingesetzt werden.

3. Verfahren zur Herstellung von Telechelen gemäß Anspruch 1, **dadurch gekennzeichnet, daß** als Komponente B Alkoxyamin-Initiatoren der Formel **I** eingesetzt werden, die - außer ihrer OH-Gruppe - keine weiteren mit Isocyanaten, Alkoholen, Carbonsäuren, Anhydriden und/oder Epoxiden reaktionsfähigen funktionellen Gruppen Y enthalten, und daß vor, während oder nach beendeter Polymerisation 1 - 5 Äquivalente der Komponente C, bezogen auf Komponente B, zugegeben werden.

4. Verfahren zur Herstellung von Telechelen gemäß Anspruch 3, **dadurch gekennzeichnet, daß** als Komponente C eine oder mehrere Verbindungen ausgewählt aus der Gruppe der Formeln

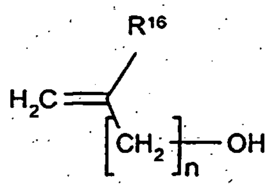
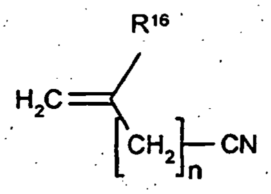
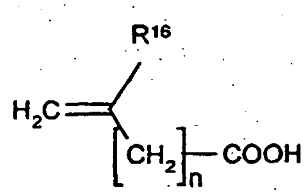

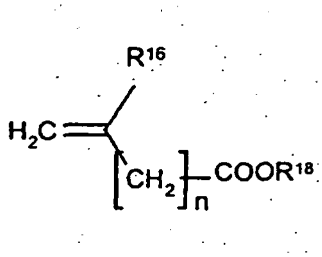
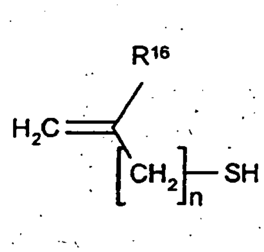
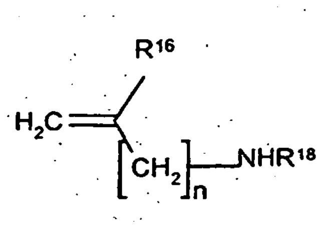

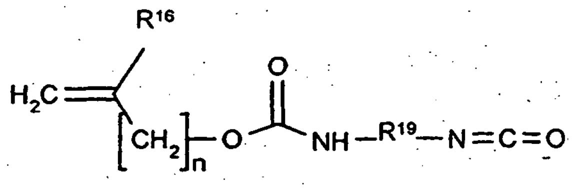

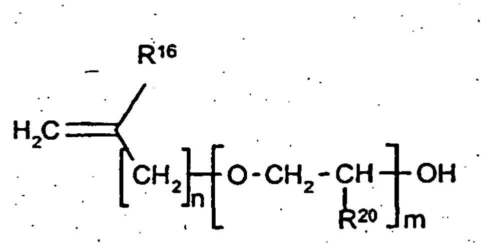

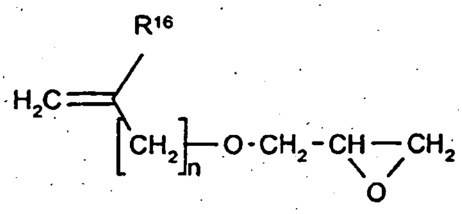

wobei
$R^{16}$ Wasserstoff oder eine lineare oder verzweigte $C_1$-$C_6$-Alkylkette und $R^{18}$ und $R^{19}$ beliebige $C_1$-$C_{24}$-Kohlenwasserstoffreste darstellen, $R^{20}$ Wasserstoff oder ein Alkylrest, bevorzugt Methyl ist, n = 1 - 4 und m = 1 - 10 sein kann, eingesetzt werden.

**5.** Verfahren zur Herstellung von Telechelen gemäß Anspruch 3, **dadurch gekennzeichnet, daß** als Komponente C eine oder mehrere Verbindungen ausgewählt aus der Gruppe 2-Propen-1-ol, 3-Buten-1-ol, 4-Penten-1-ol, 5-Hexen-1-ol oder ihrer durch Addition von 1 - 10 mol Propylenoxid an die OH-Gruppe erhaltenen propoxylierten Derivate, eingesetzt werden.

**6.** Verfahren zur Herstellung von Telechelen gemäß Anspruch 1, **dadurch gekennzeichnet, daß** als Komponente B Alkoxyamin-Initiatoren der Formel **I** eingesetzt werden, wobei mindestens einer der Reste $R^4$, $R^5$ eine mit Isocyanaten, Alkoholen, Carbonsäuren, Anhydriden und/oder Epoxiden reaktionsfähige funktionelle Gruppen Y enthält, und daß kein Funktionalisierungsreagenz C verwendet wird.

**7.** Verfahren zur Herstellung von Telechelen gemäß Anspruch 6, **dadurch gekennzeichnet, daß** als Komponente B Alkoxyamin-Initiatoren der Formel **II**b

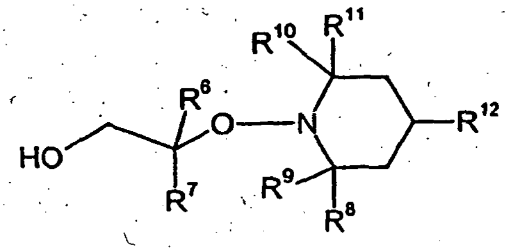

**II b**

worin
$R^6$ = H oder $CH_3$ ist, $R^7$ einen ggf. substituierten Benzolrest oder eine Estergruppe der Formel -C(=O)OR$^{13}$ darstellt, worin $R^{13}$ eine (cyclo)aliphatische Alkylgruppe mit 1 - 20 Kohlenstoffatomen ist, $R^8$, $R^9$, $R^{10}$ und $R^{11}$ unabhängig voneinander $C_1$-$C_{20}$-(Cyclo)alkyl- oder $C_6$-$C_{24}$-Arylreste bzw. $C_7$-$C_{24}$-aliphatisch/aromatische Kohlenwasserstoffreste sein können, die zusätzlich Cyanogruppen, Ethergruppen, Amidgruppen oder OH-Gruppen enthalten und auch Teil einer Ringstruktur sein können, und $R^{12}$ eine mit Isocyanaten, Alkoholen, Carbonsäuren, Anhydriden und/oder Epoxiden reaktionsfähige funktionelle Gruppe Y enthält, auf Basis von Acrylat- und Methacrylat-Monomeren eingesetzt werden.

**8.** Verfahren zur Herstellung von Telechelen gemäß Anspruch 6, **dadurch gekennzeichnet, daß** als Komponente B Alkoxyamin-Initiatoren der Formel **II c**

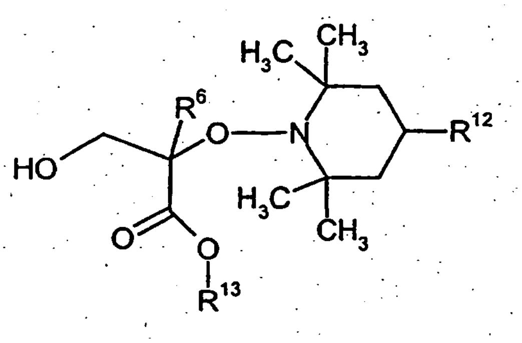

II c

worin
$R^6$ = H oder $CH_3$ ist, $R^{12}$ eine der funktionellen Gruppen OH, $NH_2$ oder NHR ist, und $R^{13}$ = $CH_3$ oder $C_4H_9$ ist, eingesetzt werden.

**9.** Telechele auf Basis von Vinyl-Polymerisaten des Molekulargewichts 200 < Mn < 50 000 und der Funktionalität von 1,5 bis 2,0 und einem Polymolekularitätsindex im Bereich von 1,1 < Mw/Mn < 1,8, enthaltend eine Hydroxyl-Endgruppe und eine mit Isocyanaten, Alkoholen, Carbonsäuren, Anhydriden und/oder Expoxiden reaktionsfähige funktionelle Gruppe Y.

**10.** Telechele gemäß Anspruch 9 der allgemeinen Formel HO-B'-Q-B" oder HO-B'-Q-C-B"' enthaltend einen gegebenenfalls substituierten Kohlenwasserstoffrest Q der Formel,

$$-\!\!\left[CHR'-CR''R'''\right]_n\!\!-$$

worin

n eine ganze Zahl im Bereich $3 \leq n \leq 500$ ist,
R', R", R"' unabhängig voneinander H, $C_1$-$C_{20}$-(Cyclo)alkyl, $C_6$-$C_{24}$-Aryl, Halogen, CN, $C_1$-$C_{20}$-Alkylester oder -amid, $C_6$-$C_{24}$-Arylester oder -amid sein können,

eine Hydroxygruppe als Endgruppe [HO-B'-], wobei B' ein Alkoxyamin-Fragment [$-CR^1-CR^2R^3-$] ist, in welchem $R^1$, $R^2$, $R^3$ unabhängig voneinander H, $C_1$-$C_{20}$-(Cyclo)alkyl, oder $C_6$-$C_{24}$-Aryl, Halogen, CN, $C_1$-$C_{20}$-(Cyclo)alkylester oder -amid, $C_6$-$C_{24}$-Arylester oder -amid können, sowie ein weiteres Alkoxyamin-Fragment [-B" bzw. -B"'] der Formel

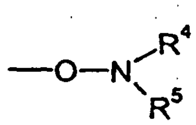

wobei
$R^4$, $R^5$ unabhängig voneinander aliphatische, cycloaliphatische oder gemischt aliphatisch/aromatische Reste mit 1 - 24 Kohlenstoffatomen, die auch Teil eines 4-8-gliedrigen Rings sein können, darstellen, worin das dem Alkoxyamin-Stickstoffatom direkt benachbarte Kohlenstoffatom der Reste $R^4$ und $R^5$ jeweils mit 3 weiteren organischen Substituenten (d.h. nicht Wasserstoff) oder einem doppelt gebundenen Kohlenstoff-, Sauerstoff-, Schwefel- oder Stickstoffatom und einem weiteren organischen Substituenten (nicht Wasserstoff) substituiert ist, und worin im Fall von B" mindestens einer der Reste $R^4$, $R^5$ eine mit Isocyanaten, Alkoholen, Carbonsäuren, Anhydriden und/oder Epoxiden reaktionsfähige funktionelle Gruppe Y enthält,
und gegebenenfalls ein Funktionalisierungsagenz C der Formel $R^{14}R^{15}C=CR^{16}(R^{17}-Y)$,

wobei
$R^{14}$, $R^{15}$, $R^{16}$ unabhängig voneinander Wasserstoff oder einen gegebenenfalls aryl-oder halogensubstuituierten Alkylrest darstellen und
$R^{17}$ eine lineare oder verzweigte, gegebenenfalls substituierte Alkylkette mit einer Mindestlänge von 1 Methylengruppe darstellt.

**11.** Kunststoffe, Klebstoffe und Fasern enthaltend Telechele gemäß Anspruch 9 oder deren Reaktionsprodukte.

**12.** Bindemittel, Härter sowie Bindemittelkomponenten und Härterkomponenten enthaltend Telechele gemäß Anspruch 9 oder deren Reaktionsprodukte.

## Claims

**1.** Process for the preparation of telechelics of the formula HO-B'-Q-B" or HO-B'-Q-C-B"' of molecular weight 500 < $M_n$ < 10,000, wherein Q represents an optionally substituted hydrocarbon radical of the formula

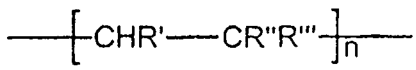

wherein
n is an integer in the range $3 \leq n \leq 500$ and wherein R', R" and R"' independently of one another can be H, $C_1$-$C_{20}$-(cyclo)alkyl, $C_6$-$C_{24}$-aryl, halogen, CN, $C_1$-$C_{20}$-(cyclo)alkyl ester or -amide or $C_6$-$C_{24}$-aryl ester or -amide, can also contain further substituents, such as ether groups, and can also be a constituent of a ring structure in a cyclic anhydride, ester, amide or hydrocarbon, **characterized in that**

A) monomers A which can be polymerized by free radicals and contain no functional group Y which is reactive with isocyanates, alcohols, carboxylic acids, anhydrides and/or epoxides, of the formula

CHR'=CR"R"'

are polymerized with
B) a functionalized alkoxyamine initiator B of the general structural formula I:

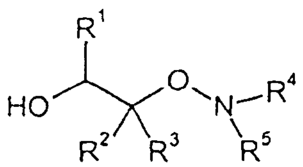

I

wherein
[-$CR^1$-$CR^2R^3$-] is represented by B,
$R^1$, $R^2$ and $R^3$ independently of one another can be H, $C_1$-$C_{20}$-(cyclo) alkyl, $C_6$-$C_{24}$-aryl, halogen, CN, $C_1$-$C_{20}$-(cyclo)alkyl ester or -amide or $C_6$-$C_{24}$-aryl ester or -amide, and
[-O-$NR^4R^5$] is represented by B" or B"',
$R^4$ and $R^5$ independently of one another represent aliphatic, cycloaliphatic or mixed aliphatic/aromatic radicals having 1 - 24 carbon atoms, which can also be part of a 4- to 8-membered ring, wherein the carbon atom of the radicals $R^4$ and $R^5$ directly adjacent to the alkoxyamine nitrogen atom is in each case substituted by 3 further organic substituents (not hydrogen) or a double-bonded carbon, oxygen, sulfur or nitrogen atom and a further organic substituent (not hydrogen), and wherein in the case of B" at least one of the radicals $R^4$ and $R^5$ contains a functional group Y which is reactive with isocyanates, alcohols, carboxylic acids, anhydrides and/or epoxides, optionally in the presence of or by subsequent addition of
C) a functionalizing reagent C of the general formula $R^{14}R^{15}C$=$CR^{16}(R^{17}$- Y), wherein $R^{17}$ represents a linear or branched, optionally substituted alkyl chain with a minimum length of 1 methylene group and wherein $R^{14}$,

$R^{15}$ and $R^{16}$ independently of one another represent hydrogen or an optionally aryl- or halogen-substituted alkyl radical.

2. Process for the preparation of telechelics according to claim 1, **characterized in that** butyl acrylate, 2-ethylhexyl acrylate, methyl methacrylate, butyl methacrylate, cyclohexyl methacrylate, isobornyl methacrylate, maleic anhydride or styrene or a mixture of such monomers are employed as component A.

3. Process for the preparation of telechelics according to claim 1, **characterized in that** alkoxyamine initiators of the formula I which - apart from their OH group - contain no further functional groups Y which are reactive with isocyanates, alcohols, carboxylic acids, anhydrides and/or epoxides are employed as component B, and **in that** 1 - 5 equivalents of component C, based on component B, are added before, during or after the end of the polymerization.

4. Process for the preparation of telechelics according to claim 3, **characterized in that** one or more compounds chosen from the group of the formulae

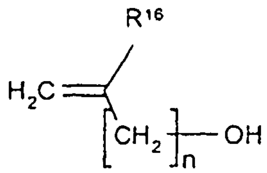
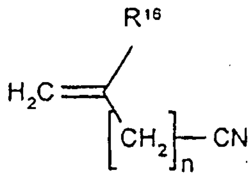
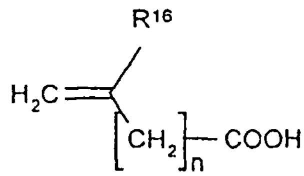

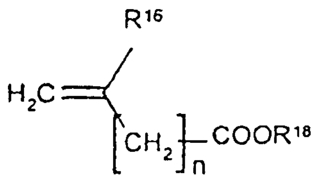
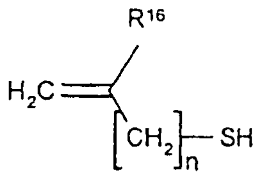
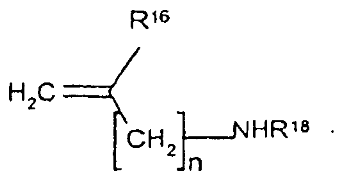

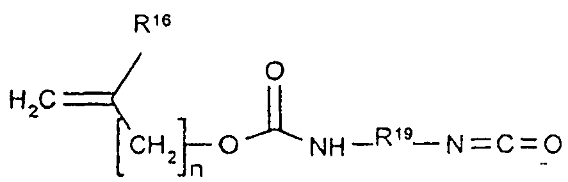
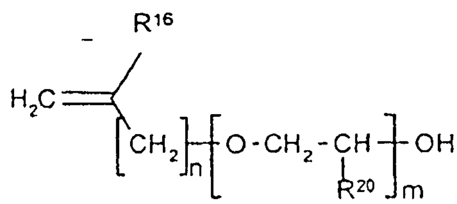

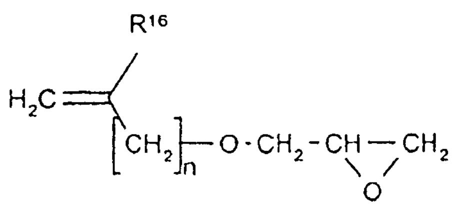

wherein
$R^{16}$ represents hydrogen or a linear or branched $C_1$-$C_6$-alkyl chain and $R^{18}$ and $R^{19}$ represent any desired $C_1$-$C_{24}$-hydrocarbon radicals, $R^{20}$ is hydrogen or an alkyl radical, preferably methyl, n can be 1 - 4 and m can be 1 - 10, are employed as component C.

**5.** Process for the preparation of telechelics according to claim 3, **characterized in that** one or more compounds chosen from the group consisting of 2-propen-1-ol, 3-buten-1-ol, 4-penten-1-ol, 5-hexen-1-ol or their propoxylated derivatives obtained by addition of 1 - 10 mol of propylene oxide on to the OH group are employed as component C.

**6.** Process for the preparation of telechelics according to claim 1, **characterized in that** alkoxyamine initiators of the formula I wherein at least one of the radicals $R^4$ and $R^5$ contains a functional group Y which is reactive with isocyanates, alcohols, carboxylic acids, anhydrides and/or epoxides are employed as component B, and **in that** no functionalizing reagent C is used.

**7.** Process for the preparation of telechelics according to claim 6, **characterized in that** alkoxyamine initiators of the formula II b

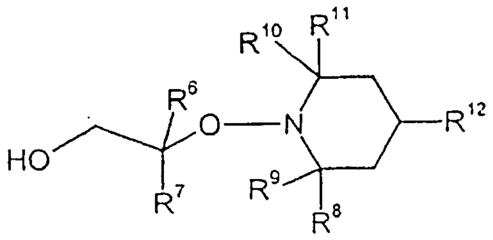

II b

wherein

$R^6$ =H or $CH_3$, $R^7$ represents an optionally substituted benzene radical or an ester group of the formula -C(=O)O$R^{13}$, wherein $R^{13}$ is a (cyclo)aliphatic alkyl group having 1 - 20 carbon atoms, $R^8$, $R^9$, $R^{10}$ and $R^{11}$ independently of one another can be $C_1$-$C_{20}$-(cyclo)alkyl or $C_6$-$C_{24}$-aryl radicals or $C_7$-$C_{24}$-aliphatic /aromatic hydrocarbon radicals, which additionally contain cyano groups, ether groups, amide groups or OH groups and can also be part of a ring structure, and $R^{12}$ contains a functional group Y which is reactive with isocyanates, alcohols, carboxylic acids, anhydrides and/or epoxides, on the basis of acrylate and methacrylate monomers, are employed as component B.

**8.** Process for the preparation of telechelics according to claim 6, **characterized in that** alkoxyamine initiators of the formula II c

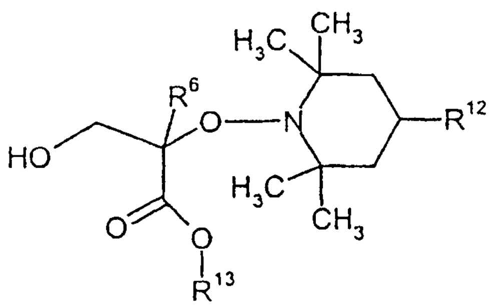

II c

wherein

$R^6$ =H or $CH_3$, $R^{12}$ is one of the functional groups OH, $NH_2$ or NHR and $R^{13}$ = $CH_3$ or $C_4H_9$, are employed as component B.

**9.** Telelchelics based on vinyl polymers of molecular weight 200 < Mn < 50,000 and of functionality of 1.5 to 2.0 and a polymolecularity index in the range of 1.1 < Mw/Mn < 1.8, containing a hydroxyl end group and a functional group Y which is reactive with isocyanates, alcohols, carboxylic acids, anhydrides and/or epoxides.

**10.** Telechelics according to claim 9 of the general formula HO-B'-Q-B" or HO-B'-Q-C-B"' containing an optionally substituted hydrocarbon radical Q of the formula

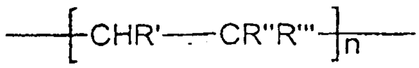

wherein

n is an integer in the range $3 \leq n \leq 500$,
R', R" and R"' independently of one another can be H, $C_1$-$C_{20}$- (cyclo) alkyl, $C_6$-$C_{24}$-aryl, halogen, CN, $C_1$-$C_{20}$-alkyl ester or -amide or $C_6$-$C_{24}$-aryl ester or -amide,

a hydroxyl group as the end group [HO-B'-], wherein B' is an alkoxyamine fragment [-$CR^1$-$CR^2R^3$-], in which $R^1$, $R^2$ and $R^3$ independently of one another can be H, $C_1$-$C_{20}$-(cyclo)alkyl, or $C_6$-$C_{24}$-aryl, halogen, CN, $C_1$-$C_{20}$-(cyclo)alkyl ester or -amide or $C_6$-$C_{24}$-aryl ester or -amide, and a further alkoxyamine fragment [-B" or -B"'] of the formula

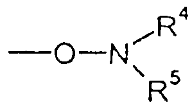

wherein
$R^4$ and $R^5$ independently of one another represent aliphatic, cycloaliphatic or mixed aliphatic/aromatic radicals having 1 - 24 carbon atoms, which can also be part of a 4- to 8-membered ring, wherein the carbon atom of the radicals $R^4$ and $R^5$ directly adjacent to the alkoxyamine nitrogen atom is in each case substituted by 3 further organic substituents (i.e. not hydrogen) or a double-bonded carbon, oxygen, sulfur or nitrogen atom and a further organic substituent (not hydrogen), and wherein in the case of B" at least one of the radicals $R^4$ and $R^5$ contains a functional group Y which is reactive with isocyanates, alcohols, carboxylic acids, anhydrides and/or epoxides,
and optionally a functionalizing agent C of the formula

$$R^{14}R^{15}C{=}CR^{16}(R^{17}\text{-}Y),$$

wherein
$R^{14}$, $R^{15}$ and $R^{16}$ independently of one another represent hydrogen or an optionally aryl- or halogen-substituted alkyl radical and
$R^{17}$ represents a linear or branched, optionally substituted alkyl chain with a minimum length of 1 methylene group.

**11.** Plastics, adhesives and fibres containing telechelics according to claim 9 or reaction products thereof.

**12.** Binders, curing agents and binder components and curing agent components containing telechelics according to claim 9 or reaction products thereof.

**Revendications**

**1.** Procédé pour la préparation de composés téléchéliques de la formule HO-B'-Q-B" ou HO-B'-Q-C-B"' du poids moléculaire $500 < M_n < 10\,000$, Q représentant un reste hydrocarboné éventuellement substitué de la formule

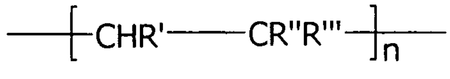

dans laquelle,
n est un nombre entier dans le domaine $3 \leq n \leq 500$ et dans laquelle R', R", R"' peuvent être indépendamment l'un de l'autre H, un groupe (cyclo)alkyle en $C_1$-$C_{20}$, aryle en $C_6$-$C_{24}$, un atome d'halogène, CN, un ester ou amide (cyclo)alkylique en $C_1$-$C_{20}$, un ester ou amide arylique en $C_6$-$C_{24}$, peuvent également contenir d'autres substituants comme des groupes éther, et peuvent également être des constituants d'une structure cyclique, dans un anhydride,

un ester, un amide ou un hydrocarbure cyclique, **caractérisé en ce que** l'on fait polymériser

A) des monomères radicalairement polymérisables A, qui ne contiennent pas de groupe Y fonctionnel capable de réagir avec des isocyanates, des alcools, des acides carboxyliques, des anhydrides et/ou des époxydes, de la formule

CHR'=CR"R"'

avec
B) un initiateur d'alcoxyamine fonctionnalisé B de la formule structurelle générale I

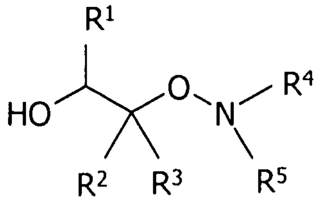

I

dans laquelle [-($R^1$-($R^2R^3$-] est représenté par B',
$R^1$, $R^2$, $R^3$ peuvent être indépendamment l'un de l'autre H, un groupe (cyclo)alkyle en $C_1$-$C_{20}$, aryle en $C_6$-$C_{24}$, un atome Le A 33 902 d'halogène, CN, un ester ou amide (cyclo)alkylique en $C_1$-$C_{20}$, un ester ou amide arylique en $C_6$-$C_{24}$, et
[-O-$NR^4R^5$] est représenté par B" respectivement B"',
$R^4$, $R^5$ représentent indépendamment l'un de l'autre des restes aliphatiques, cycloaliphatiques ou mixtes aliphatiques/aromatiques avec 1-24 atomes de carbone, lesquels peuvent également être une partie d'un noyau à 4-8 éléments, dans lequel l'atome de carbone des restes $R^4$ et $R^5$ directement voisin de l'atome d'azote de l'alcoxyamine est à chaque fois substitué avec trois autres substituants organiques (pas l'hydrogène) ou avec un atome de carbone, d'oxygène, de soufre ou d'azote doublement lié et un autre substituant organique (pas l'hydrogène), et où dans le cas de B" au moins un des restes $R^4$, $R^5$ contient un groupe Y fonctionnel capable de réagir avec des isocyanates, des alcools, des acides carboxyliques, des anhydrides et/ou des époxydes, éventuellement en présence respectivement par addition subséquente
C) d'un réactif de fonctionnalisation C de la formule générale $R^{14}R^{15}C=CR^6(R^{17}-Y)$, $R^{17}$ représentant une chaîne alkyle linéaire ou ramifiée, éventuellement substituée avec une longueur minimale de 1 groupe méthylène, et $R^{14}$, $R^{15}$ et $R^{16}$ représentant indépendamment l'un de l'autre un atome d'hydrogène ou un reste alkyle éventuellement aryle ou halogène-substitué.

**2.** Procédé pour la préparation de composés téléchéliques selon la revendication 1, **caractérisé en ce que** l'on utilise comme constituant A de l'acrylate de butyle, de l'acrylate de 2-éthylhexyle, du méthacrylate de méthyle, du méthacrylate de butyle, du méthacrylate de cyclohexyle, du méthacrylate d'isobornyle, de l'anhydride d'acide maléique ou du styrène respectivement un mélange de tels monomères.

**3.** Procédé pour la préparation de composés téléchéliques selon la revendication 1, **caractérisé en ce que** l'on utilise comme constituant B des initiateurs d'alcoxyamine de la formule I, lesquels ne contiennent - à l'exclusion de leurs groupes OH - aucun autre groupe Y fonctionnel capable de réagir avec des isocyanates, des alcools, des acides carboxyliques, des anhydrides et/ou des époxydes et **en ce que** avant, pendant ou après la polymérisation achevée 1-5 équivalents du constituant C, rapporté au constituant B, sont ajoutés.

**4.** Procédé pour la préparation de composés téléchéliques selon la revendication 3, **caractérisé en ce que** l'on utilise comme constituant C un ou plusieurs composés choisis parmi les formules

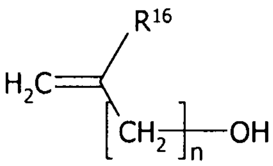
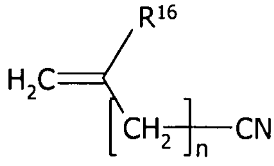
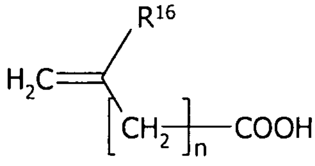

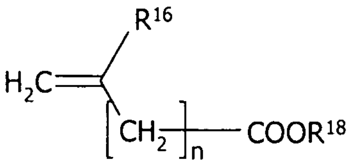
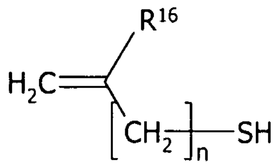
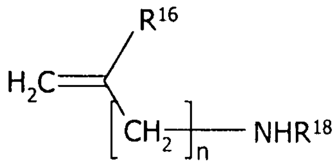

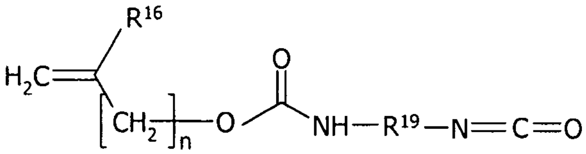
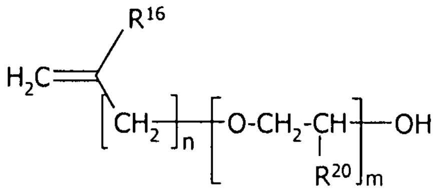

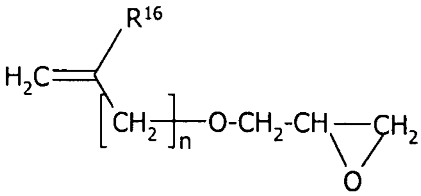

dans lesquelles
$R^{16}$ peut être un atome d'hydrogène ou une chaîne alkyle en $C_1$-$C_6$ linéaire ou ramifiée et $R^{18}$ et $R^{19}$ représentent des restes hydrocarbonés quelconques en $C_1$-$C_{24}$, $R^{20}$ peut être un atome d'hydrogène ou un reste alkyle, est de préférence le groupe méthyle, n = 1-4 et m = 1-10.

**5.** Procédé pour la préparation de composés téléchéliques selon la revendication 3, **caractérisé en ce que** l'on utilise comme constituant C un ou plusieurs composés choisis parmi le 2-propén-1-ol, le 3-butén-1-ol, le 4-pentén-1-ol, le 5-hexén-1-ol ou leurs dérivés propoxylés obtenus par addition de 1-10 mol d'oxyde de propylène sur le groupe OH.

**6.** Procédé pour la préparation de composés téléchéliques selon la revendication 1, **caractérisé en ce que** l'on utilise comme constituant B des initiateurs d'alcoxylamine de la formule I, au moins un des restes $R^4$, $R^5$ contenant un groupe fonctionnel Y capable de réagir avec des isocyanates, des alcools, des acides carboxyliques, des anhydrides et/ou des époxydes et **en ce que** l'on n'utilise aucun réactif de fonctionnalisation C.

**7.** Procédé pour la préparation de composés téléchéliques selon la revendication 6, **caractérisé en ce que** l'on utilise comme constituant B des initiateurs d'alcoxyamine de la formule IIb

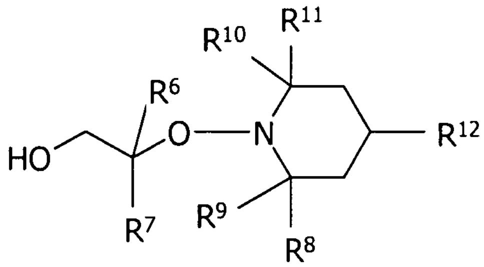

IIb

dans laquelle

$R^6$ = H ou $CH_3$, $R^7$ représente un reste benzène éventuellement substitué ou un groupe ester de la formule -C(=O)OR$^{13}$, dans laquelle $R^{13}$ est un groupe alkyle (cyclo)aliphatique avec 1-20 atomes de carbone, $R^8$, $R^9$, $R^{10}$ et $R^{11}$ peuvent être indépendamment l'un de l'autre un reste (cyclo)alkyle en $C_1$-$C_{20}$ ou aryle en $C_6$-$C_{24}$, respectivement un reste hydrocarboné aliphatique/aromatique en $C_7$-$C_{24}$ lesquels contiennent en plus des groupes cyano, des groupes éther, des groupes amide ou des groupes OH et peuvent également être une partie d'une structure cyclique, et $R^{12}$ contient un groupe Y fonctionnel capable de réagir avec des isocyanates, des alcools, des acides carboxyliques, des anhydrides et/ou des époxydes, à base de monomères d'acrylate et de méthacrylate.

**8.** Procédé pour la préparation de composés téléchéliques selon la revendication 6, **caractérisé en ce que** l'on utilise comme constituant B des initiateurs d'alcoxyamine de la formule IIc

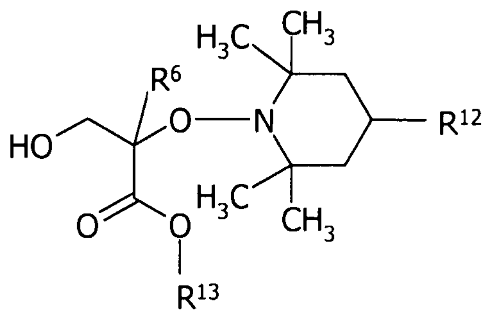

IIc

dans laquelle

$R^6$ = H ou $CH_3$, $R^{12}$ est des groupes fonctionnels OH, $NH_2$ ou NHR et $R^{13}$ = $CH_3$ ou $C_4H_9$.

**9.** Composés téléchéliques à base de polymères vinyliques du poids moléculaire 200 < Mn < 50 000 et de la fonctionnalité de 1,5 à 2,0 et d'un indice de polymolécularité dans le domaine de 1,1 < Mw/Mn < 1,8 contenant un groupe terminal hydroxyle et un groupe fonctionnel Y capable de réagir avec des isocyanates, des alcools, des acides carboxyliques, des anhydrides et/ou des époxydes.

**10.** Composés téléchéliques selon la revendication 9 de la formule générale HO-B'-Q-B" ou HO-B'-Q-C-B"' contenant un reste hydrocarboné Q facultativement substitué de la formule

$$-\left[CHR'-CR''R'''\right]_n-$$

dans laquelle,

n est un nombre entier dans le domaine $3 \leq n \leq 500$,

R', R", R"' peuvent être indépendamment l'un de l'autre H, un groupe (cyclo)alkyle en $C_1$-$C_{20}$, aryle en $C_6$-$C_{24}$, un atome d'halogène, CN, un ester ou amide alkylique en $C_1$-$C_{20}$, un ester ou amide arylique en $C_6$-$C_{24}$,

un groupe hydroxy comme groupe terminal [HO-B'-], B' étant un fragment alcoxyamine [-$CR^1$-$CR^2R^3$-], dans lequel

$R^1$, $R^2$, $R^3$ peuvent indépendamment l'un de l'autre être H, un groupe (cyclo)alkyle en $C_1$-$C_{20}$ ou aryle en $C_6$-$C_{24}$, un atome d'halogène, CN, un ester ou amide (cyclo)alkylique en $C_1$-$C_{20}$, un ester ou amide arylique en $C_6$-$C_{24}$ ainsi qu'un autre fragment d'alcoxyamine [-B" respectivement -B"'] de la formule

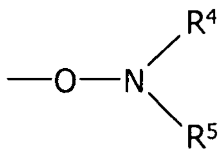

dans laquelle

$R^4$, $R^5$ peuvent être indépendamment l'un de l'autre des restes aliphatiques, cycloaliphatiques ou mixtes aliphatiques/aromatiques avec 1-24 atomes de carbone, lesquels peuvent également être une partie d'un noyau à 4-8 éléments, où l'atome de carbone des restes $R^4$ et $R^5$ directement voisin de l'atome d'azote de l'alcoxyamine est à chaque fois substitué avec trois autres substituants organiques (c'est-à-dire pas l'hydrogène) ou avec un atome de carbone, d'oxygène, de soufre ou d'azote doublement lié et un autre substituant organique (pas l'hydrogène), et où dans le cas de B" au moins un des restes $R^4$, $R^5$ contient un groupe Y fonctionnel capable de réagir avec des isocyanates, des alcools, des acides carboxyliques, des anhydrides et/ou des époxydes, et éventuellement un réactif de fonctionnalisation C de la formule $R^{14}R^{15}C=CR^{16}(R^{17}-Y)$,

dans laquelle

$R^{14}$, $R^{15}$, $R^{16}$ représentent indépendamment l'un de l'autre un atome d'hydrogène ou un reste alkyle éventuellement aryle - ou halogène - substitué et

$R^{17}$ représente une chaîne alkyle linéaire ou ramifiée, éventuellement substituée avec une longueur minimale de 1 groupe méthylène.

**11.** Matières plastiques, colles et fibres contenant des composés téléchéliques selon la revendication 9 ou leurs produits de réaction.

**12.** Liants, agents durcissants ainsi que constituants de liant et constituants d'agent durcissant contenant des composés téléchéliques selon la revendication 9 ou leurs produits de réaction.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0613910 A
- EP 0622378 A
- WO 9525765 A
- WO 9630421 A, Matyjaszewski
- WO 9718247 A
- US 4581429 A
- US 5322912 A
- US 5401804 A
- US 5412047 A
- US 5449724 A
- WO 9411412 A
- WO 9526987 A
- WO 9531484 A
- WO 9746593 A


### In der Beschreibung aufgeführte Nicht-Patentliteratur


- *Adv. Polym. Sci.,* 1987, vol. 81, 168
- **CRAIG J. HAWKER et al.** Radical Crossover in Nitroxide Mediated ''Living'' Free Radical Polymerizations. *J. Am. Chem. Soc.,* 1996, vol. 118 (46), 11467-11471
- **OTSU et al.** *Makromol. Chem., Rapid Commun,* 1982, vol. 3, 127-132
- **SAWAMOTO et al.** *Macromolecules,* 1995, vol. 282, 1721
- *Macromolecules,* 1996, vol. 29, 1070
- **PERCEC et al.** *Macromolecules,* 1995, vol. 28, 7970
- **HAWKERET.** *J.Am.Chem.Soc.,* 1994, vol. 116, 11185
- *Macromolecules,* 1995, vol. 28, 2993